# EUROPEAN PATENT APPLICATION

(11) **EP 3 933 018 A1**
(43) Date of publication of application: **05.01.2022**
(21) Application number: 20183298.7
(22) Date of filing: 30.06.2020
(51) Int. Cl.: C11D 3/48, C11D 3/20, C11D 17/00, A01N 1/02, A01N 25/02, A61Q 19/10, A61K 8/34, C11D 3/22

(54) **ANTIMICROBIAL DETERGENT COMPOSITION**

(71) Applicant: Mazzoleni, Matteo, 24022 Alzano Lombardo (BG) (IT)
(72) Inventor: Mazzoleni, Matteo, 24022 Alzano Lombardo (BG) (IT)
(74) Representative: Casciano, Lidia Giulia Rita

(57) **Abstract**

The present invention concerns a water-based antimicrobial detergent composition comprising citric acid and an alcohol selected from the group consisting of ethyl alcohol and isopropyl alcohol and the use thereof for the disinfection of a hard surface.

## Description

The present invention concerns a sanitising composition, in particular for sanitising surfaces.

In hospitals and in medical practices, it is extremely important to work in a germ-free environment when patients are being treated. For this reason, doctors, nurses and other medical staff are obliged to wash and disinfect their hands frequently. This is usually done by hand washing and cleaning, firstly using a detergent-based liquid soap followed by a second washing which involves wetting and rubbing the hands with a disinfectant composition.

Normally, the disinfectant compositions commercially available have an alcohol base and contain ethanol and/or isopropyl alcohol in a quantity higher than 60% by weight. It is generally believed in the art that these compositions require fairly high levels of ethyl alcohol in order to be antibacterial. However, since the long-term use of disinfectant compositions with high ethanol content can be associated with skin irritation, there is a need for compositions with a lower alcohol content. Furthermore, it is desirable to reduce the time and effort required for hand cleaning and disinfection in hospitals and medical practices.

Said compositions can furthermore be used also for sanitising surfaces.

EP 1 967 066 concerns an agent containing alcohol (I) that shows microbiological effectiveness within less than 30 seconds, for rapid disinfection of inanimate surfaces. The alcohol is selected from ethanol and n-propanol and iso-propanol. The agent containing alcohol also contains an amphoteric surfactant.

The object of the present invention is to provide an alcohol-based composition that has good antimicrobial and detergent properties.

Said object is achieved by means of a detergent composition according to claim 1. The preferred embodiments are indicated in the dependent claims.

In particular, a water-based antimicrobial detergent is provided comprising citric acid and an alcohol selected from the group consisting of ethyl alcohol and isopropyl alcohol, preferably ethyl alcohol.

The composition can further comprise glycerin and a thickener, preferably xanthan gum.

In a preferred embodiment, the composition of the invention comprises:
- 40 to 70% by weight of ethyl alcohol;
- 0.12 to 0.3% by weight of citric acid;
- 0.5 to 0.6% by weight of xanthan gum;
- 0.5% by weight of glycerin.

Advantageously said composition, due to the use of compounds of natural origin (hence it does not contain pesticides or petroleum derivatives), can be used several times without the risk of causing skin irritation especially in cases of delicate skin; furthermore since it is a product made of natural components it is 100% biodegradable and without any harmful impacts on the environment. Moreover, the high ethyl alcohol and isopropyl alcohol content guarantees appropriate antimicrobial properties.

In an alternative embodiment the composition of the invention, in addition to citric acid and an alcohol, also comprises acetic acid.

Preferably said composition also comprises a fragrance and more preferably comprises:
- 15% by volume of citric acid;
- 15% by volume of acetic acid;
- 40-60% by volume of ethyl alcohol;
- 0.1-0.2% by volume of limonene and
- 9.8-29.8% by volume of water.

The composition of the invention can further comprise a component selected from the group consisting of a dye and an essential oil.

The composition of the invention is applicable in disinfection of the skin.

## Claims

1. A water-based antimicrobial detergent composition comprising citric acid and an alcohol selected from the group consisting of ethyl alcohol and isopropyl alcohol.

2. The composition according to the preceding claim further comprising glycerin.

3. The composition according to any one of the preceding claims further comprising a thickener.

4. The composition according to the preceding claim **characterised in that** said thickener is xanthan gum.

5. The composition according to any one of the preceding claims **characterised in that** it comprises:
- 40 to 70% by weight of ethyl alcohol;
- 0.12 to 0.3% by weight of citric acid;
- 0.5 to 0.6% by weight of xanthan gum;
- 0.5% by weight of glycerin.

6. The composition according to claim 1 further comprising acetic acid.

7. The composition according to any one of the claims 1 or 6 comprising:
- 15% by volume of citric acid;
- 15% by volume of acetic acid;
- 40-60% by volume of ethyl alcohol;
- 0.1-0.2% by volume of limonene and
- 9.8-29.8% by volume of water.

8. The composition according to any one of the preceding claims further comprising at least a component selected from the group consisting of a dye and an essential oil.

9. The use of a composition according to any one of the claims from 1 to 8 for disinfection of the skin.
